# EUROPEAN PATENT APPLICATION

(11) **EP 4 011 445 A1**
(43) Date of publication of application: **15.06.2022**
(21) Application number: 20849297.5
(22) Date of filing: 06.08.2020
(51) Int. Cl.: A61P 3/02, A61P 5/26, A61P 13/08, A61P 15/08, A61P 15/10, A61P 15/12, A61P 19/02, A61P 21/00, A61P 25/20, A61P 25/24, A61P 35/00, A61K 36/068, A23L 31/00, A23L 33/10

(54) **COMPOSITION FOR INHIBITING REDUCTION OF TESTOSTERONE AND/OR DIHYDROTESTOSTERONE**

(30) Priority: 07.08.2019 JP 2019145513; 03.03.2020 JP 2020035899
(71) Applicant: Okinawa Ukami Sericulture Co., Ltd., Okinawa 905-0423 (JP); Tokyo University of Pharmacy & Life Sciences, Hachioji-shi, Tokyo 192-0392 (JP)
(72) Inventor: NAKASONE,Toyokazu, Kunigami-gun, Okinawa 905-0423 (JP); OKAMATSU,Shigemi, Kunigami-gun, Okinawa 905-0423 (JP); MIYAOKA,Hiroaki, Hachioji-shi, Tokyo 192-0392 (JP); TAMURA,Kazuhiro, Hachioji-shi, Tokyo 192-0392 (JP); OTA,Koichiro, Hachioji-shi, Tokyo 192-0392 (JP); YOSHIE,Mikihiro, Hachioji-shi, Tokyo 192-0392 (JP)
(74) Representative: Cabinet Beau de Loménie
(86) International application number: PCT/JP2020/030140
(87) International publication number: WO 2021/025103

(57) **Abstract**

Provided is the composition capable of effectively exerting an effect on a change in a hormone balance. A composition for inhibiting the decrease in endogenous testosterone and/or dihydrotestosterone, the composition comprising Cordyceps militaris derived from Samia cynthia ricini and/or its extract is prepared.

## Description

### TECHNICAL FIELD

The present invention relates to a composition for inhibiting the decreases in testosterone and/or dihydrotestosterone.

### BACKGROUND ART

The age around 45 to 55 years-old is called menopause, and is an age when people are more likely to experience physical changes. In this menopausal stage, the decrease in endogenous sex hormones in the body causes a change in the body. It is known that estrogen as a female ovarian hormone in women, and testosterone known as a male hormone is involved in men.

Such a change in a hormone balance and a surrounding environment influence the person to cause an uncomfortable condition and symptom, and some people are involved in life so deeply as to suffer from menopausal disorders.

In addition, changes in the hormone balance may cause the same condition and a variety of symptoms as menopausal disorders even in a younger age group, depending on the physical constitution, an environment under intense stress, not necessarily in the age of menopause.

The menopausal disorders and the related condition include the decreases in muscle strength (sarcopenia), fatigue, insomnia, joint pain, muscle pain, depression, the decreases in libido and sexual ability, and a menstrual abnormality. It is also known that disturbance of the hormone balance is associated with prostatic hypertrophy and infertility.

Improvement measures against the change in the hormone balance have been studied using various components, and for example, in Patent Document 1, attempts have been made to inhibit the decrease in blood testosterone by a plant of the genus Codonopsis or the extract .

### PRIOR ART DOCUMENT

### Patent Document

Patent Document 1: JP 2007-84559 A

### SUMMARY OF THE INVENTION

### PROBLEM TO BE SOLVED BY THE INVENTION

However, development of a component that effectively exerts an effect on a change in a hormone balance has not been sufficiently reported yet.

Although there is a technique for promoting the production of sex hormones, if their catabolism in the circulating blood cannot be inhibited, namely reduction in the decrease in the serum, it may be less likely to be effective in menopausal disorders or its related status.

### MEANS FOR SOLVING THE PROBLEM

In view of the above issue, as a result of intensive studies, the present inventors have found that Cordyceps militaris derived from Samia cynthia ricini and the extract as the component that inhibits not only a decrease in testosterone but also a decrease in dihydrotestosterone which has more potent activity, and that have completed the present invention.

That is, the invention provides the following composition.
Item 1.
   A composition for inhibiting a decrease in testosterone and/or dihydrotestosterone, the composition containing Cordyceps militaris derived from Samia cynthia ricini and/or its extract.
Item 2.
   A composition for preventing and/or treating a menopausal disorder, prostatic hypertrophy, a prostate cancer, or infertility, the composition containing Cordyceps militaris derived from Samia cynthia ricini and/or its extract.
Item 3.
   A food composition for improving a menopausal condition, a prostatic hypertrophy condition, or an infertility condition, the food composition containing Cordyceps militaris derived from Samia cynthia ricini and/or its extract.
Item 4.
   The composition according to item 2 or 3, wherein the menopausal disorder or condition is accompanied by at least one condition selected from the group consisting of a decreases in muscle strength, fatigue, insomnia, joint pain, muscle pain, depression, a menstrual abnormality, a decrease in libido, and a decrease in sexual ability.
Item 5.
   The composition according to any one of items 1 to 4, wherein the Cordyceps militaris derived from Samia cynthia ricini is obtained within 90 days from hyphal generation.
Item 6.
   The composition according to any one of items 1 to 5, wherein the composition is for oral use.
Item 7.
   The composition according to any one of items 1 to 6, wherein the composition is a food and drink, a quasi-pharmaceutical product, or a pharmaceutical product.

### EFFECT OF THE INVENTION

By applying a composition containing Cordyceps militaris derived from Samia cynthia ricini and/or its extract, the decreases in testosterone and/or dihydrotestosterone can be inhibited. In addition, it is possible to improve diseases or impaired status associated with changes in hormone balance such as male menopausal disorders, prostatic hypertrophy, or infertility.

### BRIEF DESCRIPTION OF THE DRAWINGS

Fig. 1 is the graph showing the results of the serum levels of testosterone in Test Example 1-1.
Fig. 2 is the graph showing the results of serum levels of dihydrotestosterone in Test Example 1-1.
Fig. 3 is the graph showing the results of serum levels of testosterone values in Test Example 1-1.
Fig. 4 is the graph showing the results of serum levels of dihydrotestosterone in Test Example 1-1.
Fig. 5 is the graph showing the weight of the prostate in the examination in Test Example 1-1.
Fig. 6 is the graph showing the weight of seminal vesicle in the examination in Test Example 1-1.
Fig. 7 is the graph showing changes in body weight before and after administration of the test extract in Test Example 1-1.
Fig. 8 is the graph showing the results of serum levels of testosterone in Test Example 1-2.
Fig. 9 is a graph showing the results of serum levels of dihydrotestosterone in Test Example 1-2.
Fig. 10 is the graph showing a prostate weight before and after administration of the test extract in Test Example 1-2.
Fig. 11 is the graph showing changes in body weight after the test in Test Example 1-2.
Fig. 12 is the graph showing the results of testosterone production in primary testicular cells in Test Example 2.
Fig. 13 is the graph showing the results of testosterone and dihydrotestosterone production in primary testicular cells in Test Example 2.
Fig. 14 is the graph showing the results for the viability of prostate cancer cells in Test Example 3.
Fig. 15 is the graph showing the results for the proliferation of prostate cancer cells in Test Example 3.

### MODE FOR CARRYING OUT THE INVENTION

The composition of the invention contains Cordyceps militaris derived from Samia cynthia ricini and/or its extract.

### [Cordyceps militaris derived from Samia cynthia ricini and its extract]

"Samia cynthia ricini" is different from the commonly used a silkworm phyletic line belonging to Bombycidae, and is included in the order of lepidoptera insects (silk thread insects) belonging to Saturniidae. The Samia cynthia ricini goes through the states of "egg (embryo)" (from immediately after laying eggs to immediately before hatching), "larva" (from immediately after hatching to immediately before the end of formation of a cocoon (divided into first to fifth instar stages)), "pupa" (from immediately before the end of formation of a cocoon to immediately before eclosion), and "imago (moth)" (from immediately after eclosion to death) in its lifetime, and includes any of the forms over its lifetime.

The Samia cynthia ricini alternates between the period of eating a subtropical plant and growing (instar) and the period of preparing for molting without eating (sleep) in the state of the larva after hatching from the egg. In the larva of silkworm, the period from hatching to the first molting is referred to as the first instar stage, the period from the first molting to the second molting is referred to as the second instar stage, and the fifth instar after four times of molting is usually the final instar stage. After about one week, the larva of the silkworm begins to form a cocoon by throwing up silk threads (this state is also referred to as "mature silkworm"), and pupates in the cocoon. After the pupa, the pupa ecloses to be an imago.

Cordyceps militaris is a kind of genus Cordyceps (also referred to as an insect grass fungus), and in the wild, the fungus infects a larva or a pupa of Lepidoptera, mycelia spreads in the body, and a fruiting body (mushroom part) germinates from the mycelia.

In the present specification, "Cordyceps militaris derived from Samia cynthia ricini" is not particularly limited as long as Samia cynthia ricini is used when Cordyceps militaris germinates. Typically, there is exemplified Cordyceps militaris (fruiting body) obtained by infecting Samia cynthia ricini with Cordyceps militaris. In addition, Cordyceps militaris (fruiting body) that germinates by adding Cordyceps militaris to a culture medium containing Samia cynthia ricini as a kind of nutrient component may be used.

In general, the obtained Cordyceps militaris has different kinds and blending amounts of active ingredients depending on the kind of target (host) to be infected and the nutrient component used for culture. In the invention, it has been found that it is possible to promote the production of testosterone and/or dihydrotestosterone, to improve a disease or a condition associated with a change in a hormone balance, particularly by using the Samia cynthia ricini as a host or a nutrient component.

As far as Cordyceps militaris can be infected with Cordyceps militaris or can be a nutrient component of Cordyceps militaris, the Samia cynthia ricini is not particularly limited, but preferably after the third instar stages more preferably after the fourth instar stage, further preferably after the fifth instar stage, and particularly preferably in the pupa state.

The breeding ground of the Samia cynthia ricini is not particularly limited, but it is preferably in a subtropical area, and more preferably in Okinawa and/or the Assam region of India.

The feed to be given to Samia cynthia ricini is not particularly limited, but from the viewpoint of remarkably exhibiting the effect of the invention, the feed preferably includes at least one selected from the group consisting of cassava (scientific name: Manihot esculenta) and castor-oil plant (scientific name: Ricinus communis), more preferably includes cassava and/or castor-oil plant mainly, and more preferably includes leaves of cassava and/or castor-oil plant mainly.

In addition, as the "Cordyceps militaris derived from Samia cynthia ricini" to be used, fruit bodies are preferable. The fruit body is preferably at least 1 week after germination (also referred to as hyphal generation), more preferably 10 days, still more preferably 20 days, and particularly preferably 30 days. In addition, the fruit bodies are preferably within at least 90 days from germination, more preferably within 80 days, still more preferably within 70 days, and particularly preferably within 60 days.

In addition, the form of "Cordyceps militaris derived from Samia cynthia ricini" to be used is not particularly limited as long as the effect of the invention is exhibited, but is preferably, for example, a powder or the extract.

The powder of "Cordyceps militaris derived from Samia cynthia ricini" is produced by a known method, and is obtained, for example, but not limited to, by freeze-drying the fruit bodies and then pulverizing it into a powder form.

The extract of "Cordyceps militaris derived from Samia cynthia ricini" is produced by a known method, and is not particularly limited. Examples of the solvent used for extraction include water and/or an organic solvent, and examples of the solvent include organic solvents such as methanol, ethanol, acetonitrile, acetone, chloroform, dioxane, tetrahydrofuran, isopropyl alcohol, n-hexane, and ethyl acetate. The extraction solvent is preferably water (including hot water) and/or ethanol. As the extraction solvent, one kind or two or more kinds of the solvents and other known solvents can be appropriately used in combination.

The obtained extract liquid can be appropriately subjected to treatment such as purification, freezing, and drying, and concentrated and dried to obtain a solid extract.

### [Application (Use)]

Since the composition of the invention contains Cordyceps militaris derived from Samia cynthia ricini and/or its extract, it is possible to inhibit the reduction in testosterone and/or dihydrotestosterone.

In men, testosterone is mainly secreted from the interstitial cells of the testis. This hormone is secreted from the ovary or adrenal gland in women, whose levels are 5 to 10% of those in men. Testosterone is converted to biologically most potent androgen dihydrotestosterone by 5α reductase and exerts various physiological activities. The testosterone and dihydrotestosterone are catabolized in many body tissues and lose their activity.

There are various physiological activities in which testosterone is involved, and for example, an action of maintaining muscle mass and strength, the actions of reducing fatigue, of improving sleep, joint pain, and muscle pain, depression, menstrual abnormality, libido, sexual ability, and concentrated force, an action of reducing a visceral fat, an action of improving a cognitive function and bone density are known ("Male Menopausal Disorder (LOH Syndrome)" described by Shigeo Horie, Journal of the Japanese Society of Internal Medicine, Vol. 102, No. 4, 2013). The present invention implies that it could be possible to enhance the physiological activity involving testosterone by the inhibition of the decrease in testosterone in the body.

Changes in hormone balance including the decrease in sex hormones occur due to aging and environmental stressors. After around 45 years old, it becomes the stage called menopause, and in that age (also referred to as fluctuation generation), people are more likely to suffer from socalled menopausal disorders and the related conditions and are more likely to feel physical changes. It is generally accepted that the decreases in the serum levels of estrogen known as a female hormone and of testosterone known as a male hormone are important issues.

In the present specification, the menopausal disorders refers to "among a wide variety of symptoms that appear in middle-aged male and female, the symptoms that is not caused by structural changes in organs, but interfere with daily life are referred to as menopausal symptoms. Further, the menopausal condition, menopausal-related status, is designated as the condition in which at least a part of the menopausal symptoms are exhibited or at least a part of the menopause symptoms are of concern, although their daily life is not interfered.

In the present specification, the menopause symptom is referred to as "among a wide variety of symptoms that appear in menopause, the symptoms that are not accompanied with structural changes in various organs", and basically is divided into autonomic ataxia symptoms mainly including a feeling of heat, hot flashes, heart palpitations, sweating, and insomnia, and psychoneurotic symptoms mainly including anxiety, depression, a feeling of fear, and fatigue, and also includes a case accompanied by the decreases in muscle strength, joint pain, muscle pain, menstrual abnormality, the decreases in libido and sexual ability.

The composition containing Cordyceps militaris derived from Samia cynthia ricini and/or an its extract exhibits the effect of inhibiting the decreases in sex hormones, and thus effectively acts on menopausal disorders or the menopausal condition, and is effective for at least one selected from the group consisting of a decrease in muscle strength, fatigue, insomnia, joint pain, muscle pain, depression, menstrual abnormality, the decreases in libido and sexual ability.

It has been known that excessive dihydrotestosterone production has a negative effect on the functions of hair growth and the prostate glands. However, when the levels of dihydrotestosterone decreases, growth as a male in the embryonic stage and reproductive/gonadal functions in the adult are inhibited, so that the decrease also becomes a problem.

In addition, according to recent reports, testosterone and dihydrotestosterone are also produced in the hippocampus, and it has been found that these hormones act directly to increase nerve synapses and spines (Suguru Kawato, "Male and Female Hormones Synthesized in Brain's Hippocampus Enhance Memory", Journal of Japanese Biochemical Society 88(3) : 342-353 (2016)). It is suggested that when testosterone or dihydrotestosterone decreases in the brain due to aging or the like, the number of spines decreases, a memory function decreases, and this may lead to dementia or Alzheimer's disease. Therefore, by inhibiting the decrease in testosterone and/or dihydrotestosterone, it is also effective for improving, preventing, or treating the memory function, dementia, or Alzheimer's disease.

In addition, in Examples described later, it has been found that a composition containing Cordyceps militaris derived from Samia cynthia ricini and/or its extract possess the weight reducing effects after its continuous treatment. Therefore, it is possible to effectively use Cordyceps militaris derived from Samia cynthia ricini and/or its extract for anti-obesity and inhibition of increased fat mass.

In addition, in Examples described as below, it has been found that the composition comprising Cordyceps militaris derived from Samia cynthia ricini and/or an its extract inhibits prostatic hypertrophy. Therefore, it is possible to effectively use Cordyceps militaris derived from Samia cynthia ricini and/or its extract for prevention and/or treatment of prostatic hypertrophy. Furthermore, when prostatic hypertrophy is inhibited, urination is performed normally, and thus it is expected that the amount of urine accumulated in the bladder is reduced and the frequent urination condition is improved. Therefore, it is possible to effectively use Cordyceps militaris derived from Samia cynthia ricini and/or its extract for improving a urination disorder and/or frequent urination.

In addition, in Examples described later, it has been found that the composition containing Cordyceps militaris derived from Samia cynthia ricini and/or its extract inhibits proliferation of a prostate cancer cell. Therefore, it is possible to effectively use Cordyceps militaris derived from Samia cynthia ricini and/or its extract for prevention and/or treatment of prostate cancer.

In addition, although data is not shown, it has also been found that the diuretic action is enhanced by administration of the composition containing Cordyceps militaris derived from Samia cynthia ricini and/or its extract, to a prostatic hypertrophy model rat and mouse used in Examples as described below. By enhancing the diuretic action, it is expected to promote sodium excretion, leading to improvement of swelling. Therefore, it is possible to effectively use Cordyceps militaris derived from Samia cynthia ricini and/or its extract for improvement of the swelling.

The composition of the invention can further appropriately contain an active ingredient or an additive that can be used for a food and drink, a food labeled with a functionality, a specified health food, a quasi-pharmaceutical product, and a pharmaceutical product, and can be appropriately formulated by a known formulation method used for the item.

### (Formulation)

In the invention, the composition of the invention can also be administered orally (oral preparation) or parenterally as solid formulations such as a tablet, a capsule, a granule, or a powder; or as liquid formulations such as a syrup or an injection. An excipient, a lubricant, a binder, and a disintegrant as the solid formulations, and a solvent, a solubilizing agent, a suspending agent, a tonicity agent, a buffering agent, and a soothing agent as the liquid formulation can be used. If necessary, additives such as a preservative, an antioxidant, a colorant, and a sweetening agent can also be used.

Examples of the excipient include sugar alcohols such as D-sorbitol, mannitol, and xylitol; saccharides such as glucose, white sugar, lactose, and fructose; crystalline cellulose, carmellose sodium, croscarmellose sodium, calcium hydrogen phosphate, wheat starch, rice starch, corn starch, potato starch, dextrin, β-cyclodextrin, light anhydrous silicic acid, titanium oxide, magnesium aluminate metasilicate, talc, kaolin, and an olive oil.

Examples of the binder include cellulose derivatives such as methyl cellulose, ethyl cellulose, hydroxypropyl cellulose, and hydroxypropyl methyl cellulose; polyvinyl pyrrolidone, polyvinyl alcohol, an acrylic acid-based polymer, gelatin, gum arabic, pullulan, pregelatinized starch, agar, tragacanth, sodium alginate, and propylene glycol alginate.

Examples of the disintegrant include starch, a low substituted hydroxypropyl cellulose, carboxymethyl cellulose calcium, croscarmellose sodium, hydroxypropyl starch, and partially pregelatinized starch.

Examples of the solvent include a water for injection, an alcohol, propylene glycol, macrogol, a sesame oil, and a corn oil.

Examples of the lubricant include stearic acid, magnesium stearate, calcium stearate, polyoxyl stearate, cetanol, talc, a hardened oil, sucrose fatty acid ester, dimethylpolysiloxane, beeswax, and white beeswax.

Examples of the solubilizing agent include polyethylene glycol, propylene glycol, D-mannitol, benzyl benzoate, ethanol, trisaminomethane, cholesterol, triethanolamine, sodium carbonate, and sodium citrate.

Examples of the suspending agent/emulsifier include surfactants such as stearyltriethanolamine, sodium lauryl sulfate, laurylaminopropionic acid, lecithin, benzalkonium chloride, benzethonium chloride, and glyceryl monostearate; hydrophilic polymers such as polyvinyl alcohol, polyvinylpyrrolidone, sodium carboxymethylcellulose, methylcellulose, hydroxymethylcellulose, hydroxyethylcellulose, and hydroxypropylcellulose; waxes such as shellac wax, beeswax, carnauba wax, spermaceti wax, lanolin, liquid lanolin, reduced lanolin, hard lanolin, cyclic lanolin, lanolin wax, candelilla wax, moth wax, montan wax, ceramic wax, and rice wax.

Examples of the tonicity agent include sodium chloride, glycerin, and D-mannitol.

Examples of the buffering agent include buffers such as phosphate, acetate, carbonate, and citrate.

Examples of the preservative include paraoxybenzoic acid esters, chlorobutanol, benzyl alcohol, phenethyl alcohol, dehydroacetic acid, and sorbic acid.

Examples of the antioxidant include sulfite and an ascorbic acid.

When the composition of the invention is formed into the solid formulation, a production method known in the art can be used. Examples of the method include a method of pulverizing and sizing an extruded granulated product molded by kneading the composition and passing the kneaded composition through a screen, and a method of adding water for kneading to the composition, stirring and granulating by molding with a vertical granulator, and then pulverizing and sieving using a comil. In addition, a method in which the formulation composition is compressed with a roller compactor, then pulverized with a roll granulator, and sieved may be mentioned. Further, a method in which fluidized bed drying is performed after stirring and granulation can be mentioned. In addition, for example, in the case of producing by direct compression, the composition may be mixed and then directly put into a tablet pressing machine to be compressed.

### (Food and drink)

The composition of the invention can also be used as a food and drink composition, and can be provided by being contained in a food or functional food. Examples of such a food or functional food include rice; various noodles such a buckwheat noodle, a Japanese wheat noodle, a bean-starch vermicelli (harusame), a Chinese noodle, an instant noodle, and a pot noodle; beverages such as a soft drink, a carbonated drink, a nutritional drink, a fruit drink, a lactic acid drink, and a sports drink; various soups such as curry roux, a stew; frozen confectioneries such as an ice cream, an ice sherbet, and a shaved ice; confectioneries such as a candy, a cookie, a candy, a gum, chocolate, a tablet confectionery, a snack, a biscuit, jelly, jam, cream, and other baked confectionery; fish and livestock processed foods such as boiled fish paste (kamaboko), minced fish (hanpen), ham, and sausage; dairy products such as a processed milk and a fermented milk; fats and oils, and fat-and-oil processed foods such as a salad oil, a fritter oil, margarine, mayonnaise, shortening, whipped cream, and dressing; condiments such as sauce, dressing, bean paste (miso), soy sauce, and sauce; a soup, a stew, a salad, a side dish, a dried food sprinkled over rice (furikake), a pickled vegetable; various other forms of a health and food supplement, a food labeled with a functionality, and a specified health food.

Furthermore, a supplement (a powder, a granule, a soft capsule, a hard capsule, a tablet, a chewable tablet, a fast dissolving tablet, syrups, jelly, and a liquid) containing the composition of the invention may be prepared.

The composition of the invention can also be contained in a feed for an animal such as a pet.

The additive is added to the food and drink as necessary. Examples of such an additive include glucose, fructose, sucrose, maltose, sorbitol, stevioside, rebaudioside, corn syrup, lactose, mannitol, dextrin, citric acid, sodium citrate, tartaric acid, malic acid, succinic acid, lactic acid, L-ascorbic acid, dl-α-tocopherol, sodium erythorbate, glycerin, propylene glycol, glycerin fatty acid ester, polyglycerin fatty acid ester, sucrose fatty acid ester, sorbitan fatty acid ester, gum arabic, carrageenan, casein, gelatin, pectin, agar, vitamin C, vitamin B, vitamin E, nicotinic acid amide, calcium pantothenate, amino acids, calcium salts, surfactant, dye, flavor, and preservative.

The composition of the invention can be used for the food and drink that is permitted to display improvement/prevention of various symptoms and conditions. Here, in the invention, the food and drink that is permitted to display improvement/prevention of various symptoms and conditions is a food and drink having pharmaceutical efficacy permitted/designated by the country or public organization, and is, for example, a food labeled with a functionality, a food with health claims such as a specified health food, and a food and drink for special use. Note that the name and stipulation vary depending on the situation, the times, and the system of each country, but those that are essentially the same are included in the invention.

The blending amount of the composition of the invention is not particularly limited, and is appropriately set according to the purpose of application (kind of a target disease or symptom), an application target site, sex and age of the application user, a form of a food and drink, food labeled with a functionality, specified health food, quasi-pharmaceutical product, or pharmaceutical product, a method of administering or ingesting these, the number of administrations/ingestions, and preference.

When the composition of the invention is used, the daily intake of Cordyceps militaris derived from Samia cynthia ricini or its extract can be, for example, 0.005 to 4,000 mg, preferably 0.1 to 3,000 mg, more preferably 0.5 to 2,000 mg, still more preferably 1 to 1,000 mg, particularly preferably 2 to 500 mg, and most preferably 3 to 300 mg in terms of dry solid content.

The period of intake of the composition of the invention is not particularly limited, and is appropriately set according to the purpose of application (kind of a target disease or symptom), an application target site, sex and age of the application user, a form of a food and drink, food labeled with a functionality, specified health food, quasi-pharmaceutical product, or pharmaceutical product. The period of intake of the composition of the invention can be, for example, 1 day or longer, and is preferably 7 days or longer, more preferably 12 days or longer, and still more preferably 1 month or longer.

The dosage form of the composition of the invention is not particularly limited in the formulation. For example, functional foods such as a supplement and a nutritional drink, confectioneries such as a gummi candy, a candy, gum, a cracker, and a biscuit; foods such as an ice cream, jelly, sweet beans jelly (yokan), a bread, steamed egg hotchpotch (chawan-mushi), and curry; beverages such as a milk beverage, a soft drink, and a soup; condiments such as a dressing and ponzu sauce are exemplified as a food and drink composition that can be consumed.

Although not limited, the composition of the invention is preferably ingested as the functional food from the viewpoint of the effect of the invention, and examples of the functional food include a food labeled with a functionality, a food with nutrient function claims, a food supplement, and a specified health food, and the food labeled with a functionality is preferable in that the use can be clearly displayed.

### [Subject applied]

The subject to which the composition of the invention is applied is not particularly limited, but may be around 30 years old or older, who is an age susceptible to stress from living environment such as work, may be middle-aged people (around 45 years old or older and under 55 years old), who are an age susceptible to a change in the body due to a change in a hormone balance, or may be elderly people (55 years old or older). From the viewpoint of an age susceptible to a change in the body due to a change in a hormone balance, the composition of the invention is preferably applied to middle-aged and elderly people (around 40 years old or older, around 45 years old or older, around 50 years old or older, and around 55 years old or older).

### [pH]

The pH of the composition of the invention is appropriately set according to the kind and content of other blending components, the formulation form, and the use method, and is not limited as long as it is within a physiologically or pharmaceutically acceptable range, and can be, for example, pH 2 to 9. From the viewpoint of stably exhibiting the effect of the invention, the pH of the composition of the invention can be preferably 2 to 9.5, more preferably 3 to 9, still more preferably 4 to 8.5, still more preferably 4.5 to 8, and still more preferably 5.5 to 7.5.

### [Embodiment]

Regarding the embodiments, but not limited to, the invention discloses the following embodiments.

A composition for inhibiting the decreases in testosterone and/or dihydrotestosterone, the composition containing Cordyceps militaris derived from Samia cynthia ricini and/or its extract.

The composition for preventing and/or treating menopausal disorders, prostatic hypertrophy, prostate cancer, or infertility, the composition containing Cordyceps militaris derived from Samia cynthia ricini and/or its extract.

A food composition for improving menopausal conditions, a prostatic hypertrophy condition, or an infertility condition, the food composition containing Cordyceps militaris derived from Samia cynthia ricini and/or its extract.

The composition, wherein the menopausal disorders or condition is accompanied by at least one condition selected from the group consisting of the decreases in muscle strength, fatigue, insomnia, joint pain, muscle pain, depression, menstrual abnormality, and the decreases in libido and sexual ability.

The composition for improving sex growth in the embryonic stage, the composition containing Cordyceps militaris derived from Samia cynthia ricini and/or the extract .

A composition for improving memory functions, dementia, or Alzheimer's disease, the composition containing Cordyceps militaris derived from Samia cynthia ricini and/or the extract .

The composition for improving obesity, containing Cordyceps militaris derived from Samia cynthia ricini and/or the extract .

The composition for improving prostatic hypertrophy, a urination disorder, or frequent urination, the composition containing Cordyceps militaris derived from Samia cynthia ricini and/or the extract.

The composition for preventing and/or treating a prostate cancer, the composition containing Cordyceps militaris derived from Samia cynthia ricini and/or the extract .

The composition, wherein the Samia cynthia ricini is bred on feed mainly containing cassava and/or castor-oil plant.

The composition, wherein the Samia cynthia ricini is bred on feed including leaves of cassava and/or castor-oil plant mainly.

The composition, wherein the Samia cynthia ricini has been infected with Cordyceps militaris in the pupa state.

The composition, wherein the Cordyceps militaris derived from Samia cynthia ricini is obtained within 90 days from hyphal generation.

The composition, wherein the extract of Cordyceps militaris derived from Samia cynthia ricini is extracted with water and/or an organic solvent.

The composition, wherein the organic solvent is at least one selected from the group consisting of methanol, ethanol, acetonitrile, acetone, chloroform, dioxane, tetrahydrofuran, isopropyl alcohol, n-hexane, and ethyl acetate.

The composition being for oral use.

The composition being an oral preparation.

The composition being a tablet, a capsule, a granule, a powder, or a syrup.

The composition being a functional food, a food and drink, a quasi-pharmaceutical product, or a pharmaceutical product.

The composition for use in a person of 40 years old or more, 45 years old or more, 50 years old or more, or 55 years old or more.

Cordyceps militaris derived from Samia cynthia ricini and/or its extract for use in inhibiting the reduction in testosterone and/or dihydrotestosterone.

Cordyceps militaris derived from Samia cynthia ricini and/or its extract for use in prevention and/or treatment of menopausal disorders, prostatic hypertrophy, prostate cancer or infertility.

Cordyceps militaris derived from Samia cynthia ricini and/or its extract for use in improving menopausal condition, a prostatic hypertrophy condition, or an infertility condition.

The Cordyceps militaris derived from Samia cynthia ricini and/or the extract , wherein the menopausal disorders or condition is accompanied by at least one condition selected from the group consisting of the decrease in muscle strength, fatigue, insomnia, joint pain, muscle pain, depression, menstrual abnormality, and decreases in libido and sexual ability.

Cordyceps militaris derived from Samia cynthia ricini and/or its extract for use in improving sexual maturation in the embryonic stage.

Cordyceps militaris derived from Samia cynthia ricini and/or its extract for use in improving a memory function, dementia, or Alzheimer's disease.

Cordyceps militaris derived from Samia cynthia ricini and/or its extract for use in improving obesity.

Cordyceps militaris derived from Samia cynthia ricini and/or the extract for use in improving prostatic hypertrophy, a urination disorder, or frequent urination.

Cordyceps militaris derived from Samia cynthia ricini and/or the extract ricini for use in preventing and/or treating a prostate cancer.

The Cordyceps militaris derived from Samia cynthia ricini and/or the extract , wherein the Samia cynthia ricini is bred on feed mainly including cassava and/or castor-oil plant.

The Cordyceps militaris derived from Samia cynthia ricini and/or the extract , wherein the Samia cynthia ricini is bred on feed including leaves of cassava and/or castor-oil plant mainly.

The Cordyceps militaris derived from Samia cynthia ricini and/or its extract , wherein the Samia cynthia ricini has been infected with Cordyceps militaris in the pupa state.

The Cordyceps militaris derived from Samia cynthia ricini and/or the extract , wherein the Cordyceps militaris derived from Samia cynthia ricini is obtained within 90 days from hyphal generation.

The Cordyceps militaris derived from Samia cynthia ricini and/or its extract, wherein the extract of Cordyceps militaris derived from Samia cynthia ricini is extracted with water and/or an organic solvent.

The Cordyceps militaris derived from Samia cynthia ricini and/or its extract, wherein the organic solvent is at least one selected from the group consisting of methanol, ethanol, acetonitrile, acetone, chloroform, dioxane, tetrahydrofuran, isopropyl alcohol, n-hexane, and ethyl acetate.

The Cordyceps militaris derived from Samia cynthia ricini and/or the extract being for oral use.

The Cordyceps militaris derived from Samia cynthia ricini and/or the extract being an oral preparation.

The Cordyceps militaris derived from Samia cynthia ricini and/or the extract being a tablet, a capsule, a granule, a powder, or a syrup.

The Cordyceps militaris derived from Samia cynthia ricini and/or the extract being a functional food, a food and drink, a quasi-pharmaceutical product, or a pharmaceutical product.

The Cordyceps militaris derived from Samia cynthia ricini and/or the extract for use in a person of 40 years old or more, 45 years old or more, 50 years old or more, or 55 years old or more.

The Cordyceps militaris derived from Samia cynthia ricini and/or its extract, wherein the use is a nontherapeutic use.

A use for producing a composition for inhibiting the decrease in testosterone and/or dihydrotestosterone, the composition containing Cordyceps militaris derived from Samia cynthia ricini and/or its extract .

A use for producing a composition for preventing and/or treating menopausal disorders, prostatic hypertrophy, prostate cancer, or infertility, the composition containing Cordyceps militaris derived from Samia cynthia ricini and/or its extract .

A use for producing the composition for improving a menopausal condition, a prostatic hypertrophy condition, or an infertility condition, the composition containing Cordyceps militaris derived from Samia cynthia ricini and/or its extract .

The use, wherein menopausal disorders or condition is accompanied by at least one condition selected from the group consisting of the decrease in muscle strength, fatigue, insomnia, joint pain, muscle pain, depression, the decreases in libido and sexual ability, and a menstrual abnormality.

A use for producing the composition for improving sexual maturation in the embryonic stage, the composition containing Cordyceps militaris derived from Samia cynthia ricini and/or its extract.

A use for producing the composition for improving memory functions, dementia, or Alzheimer's disease, the composition containing Cordyceps militaris derived from Samia cynthia ricini and/or its extract.

A use for producing the composition for improving obesity, the composition containing Cordyceps militaris derived from Samia cynthia ricini and/or its extract.

A use for producing the composition for improving prostatic hypertrophy, dysuria such as frequent urination and residual urine, the composition containing Cordyceps militaris derived from Samia cynthia ricini and/or the extract.

A use for producing the composition for preventing and/or treating a prostate cancer, the composition containing Cordyceps militaris derived from Samia cynthia ricini and/or its extract .

The use, wherein the Samia cynthia ricini is bred on feed mainly containing cassava and/or castor-oil plant.

The use, wherein the Samia cynthia ricini is bred on feed including leaves of cassava and/or castor-oil plant mainly.

The use, wherein the Samia cynthia ricini has been infected with Cordyceps militaris in the pupa state.

The use, wherein the Cordyceps militaris derived from Samia cynthia ricini is obtained within 90 days from hyphal generation.

The use, wherein the extract of Cordyceps militaris derived from Samia cynthia ricini is extracted with water and/or an organic solvent.

The use, wherein the organic solvent is at least one selected from the group consisting of methanol, ethanol, acetonitrile, acetone, chloroform, dioxane, tetrahydrofuran, isopropyl alcohol, n-hexane, and ethyl acetate.

The use, wherein the composition is for oral use.

The use, wherein the composition is an oral preparation.

The use, wherein the composition being a tablet, a capsule, a granule, a powder, or a syrup.

The use being a functional food, a food and drink, a quasi-pharmaceutical product, or a pharmaceutical product.

The use for use in a person of 40 years old or more, 45 years old or more, 50 years old or more, or 55 years old or more.

A method of inhibiting the decrease in testosterone and/or dihydrotestosterone, comprising administration of Cordyceps militaris derived from Samia cynthia ricini and/or its extract to humans.

A method of preventing and/or treating a menopausal disorder, prostatic hypertrophy, a prostate cancer, or infertility, comprising administration of Cordyceps militaris derived from Samia cynthia ricini and/or the extract to humans.

A method of improving a menopausal condition, a prostatic hypertrophy condition, or an infertility condition, comprising administration of Cordyceps militaris derived from Samia cynthia ricini and/or the extract to humans.

The method, wherein the menopausal disorders or condition is accompanied by at least one condition selected from the group consisting of the decrease in muscle strength, fatigue, insomnia, joint pain, muscle pain, depression, menstrual abnormality, and the decreases in libido and a sexual ability.

A method of improving sex growth in an embryonic stage, comprising administration of Cordyceps militaris derived from Samia cynthia ricini and/or the extract to humans.

A method of improving memory function, dementia, or Alzheimer's disease, comprising administration of Cordyceps militaris derived from Samia cynthia ricini and/or the extract to humans.

A method of improving obesity, comprising administration of Cordyceps militaris derived from Samia cynthia ricini and/or the extract to humans.

A method of improving prostatic hypertrophy, a urination disorder, or frequent urination, comprising administration of Cordyceps militaris derived from Samia cynthia ricini and/or the extract to humans.

A method of preventing and/or treating prostate cancers, comprising administration of Cordyceps militaris derived from Samia cynthia ricini and/or the extract to humans.

The method, wherein the Samia cynthia ricini is bred on feed mainly containing cassava and/or castor-oil plant.

The method, wherein the Samia cynthia ricini is bred on feed including leaves of cassava and/or castor-oil plant mainly.

The method, wherein the Samia cynthia ricini has been infected with Cordyceps militaris in the pupa state.

The method, wherein the Cordyceps militaris derived from Samia cynthia ricini is obtained within 90 days from hyphal generation.

The method, wherein the extract of Cordyceps militaris derived from Samia cynthia ricini is extracted with water and/or an organic solvent.

The method, wherein the organic solvent is at least one selected from the group consisting of methanol, ethanol, acetonitrile, acetone, chloroform, dioxane, tetrahydrofuran, isopropyl alcohol, n-hexane, and ethyl acetate.

The method including orally ingesting the composition.

The method including ingesting the composition as an oral preparation.

The method including ingesting the composition as tablet, a capsule, a granule, a powder, or a syrup.

The method including ingesting the composition as a functional food, a food and drink, a quasi-pharmaceutical product, or a pharmaceutical product.

The method for use in a person of 40 years old or more, 45 years old or more, 50 years old or more, or 55 years old or more.

### EXAMPLES

Next, the invention is specifically described with reference to Examples and Test Examples, but the invention is not limited to the following Examples and Test Examples.

### (Preparation of Cordyceps militaris derived from Samia cynthia ricini)

A pupa of Samia cynthia ricini (Okinawa UKAMI Sericulture Co., Okinawa prefecture, bred on feed including leaves of cassava (scientific name: Manihot esculenta) and/or castor-oil plant (scientific name: Ricinus communis) mainly was infected with Cordyceps militaris and cultured for 50 days in a dark room at 25°C. The resulting fruiting body was frozen and dried.

### (Preparation 1 of hot water extract)

A hot water extract 1 was obtained by adding 300 mL of a purified water to 7.7 g of the obtained dried fruiting body to obtain a mixture and heating the mixture at 100°C for 24 hours. The hot water extract 1 was collected by suction filtration with a qualitative filter paper (No. 2 manufactured by ADVANTEC Corporation). A hot water extract 2 was obtained by adding 300 mL of a purified water to the filtered residue to obtain a mixture and heating the mixture at 100°C for 24 hours. The hot water extract 2 was collected by suction filtration with the qualitative filter paper (No. 2 manufactured by ADVANTEC Corporation). The hot water extracts 1 and 2 were combined and concentrated with a solvent concentrator (EYELA DPE-1150, manufactured by TOKYO RIKAKIKAI CO, LTD.) and a rotary evaporator (EYELA N-1210BVF-W, manufactured by TOKYO RIKAKIKAI CO., LTD.). The concentrated hot water extract was freeze-dried in a freeze-dryer (EYELA FDU-1200, TOKYO RIKAKIKAI) to obtain a powder of the hot water extract.

The resulting powder of the hot water extract had a dry weight of 5.8 g and a yield of 75% with respect to 7.7 g of the dried fruiting body.

### (Preparation 2 of hot water extract)

A hot water extract 1 was obtained by crushing 10.9 g of the dried fruiting body to obtain a fruiting body powder, adding 400 mL of a purified water to the powder to obtain a mixture, and heating the mixture at 100 °C for 36 hours. The hot water extract 1 was centrifuged at 3800/rpm, 23°C for 10 minutes, and the supernatant was collected by suction filtration with a filter paper (No. 5A manufactured by Kiriyama Glass Works Co.). A hot water extract 2 was obtained by adding 400 mL of a purified water to the filtered residue to obtain a mixture and heating the mixture at 100 °C for 24 hours. The hot water extract 2 was centrifuged at 3800/rpm, 23°C for 10 minutes, and the supernatant was collected by suction filtration with a filter paper (No. 5A manufactured by Kiriyama Glass Works Co.). The hot water extracts 1 and 2 were combined and concentrated with a solvent concentrator (EYELA DPE-1150, manufactured by TOKYO RIKAKIKAI CO, LTD.) and a rotary evaporator (EYELA N-1210BVF-W, manufactured by TOKYO RIKAKIKAI CO., LTD.). The concentrated hot water extract was freeze-dried in the freeze-dryer (EYELA FDU-1200, manufactured by TOKYO RIKAKIKAI CO., LTD.) to obtain a powder of the hot water extract.

The resulting powder of the hot water extract had a dry weight of 8.4 g and a yield of 77% with respect to 10.9 g of the dried fruiting body.

### (Preparation of organic solvent extract and hot water extract)

An n-hexane extract 1 was obtained by adding 100 mL of n-hexane to 4.8 g of the obtained dried fruiting body and allowing it to stand at 23°C for 24 hours. The supernatant was collected by a gradient method, 100 mL of n-hexane was then added to the extraction residue, and the mixture was allowed to stand at room temperature for 24 hours to obtain an n-hexane extract 2. The supernatant was collected by the gradient method, 100 mL of n-hexane was then added again, and the mixture was allowed to stand at room temperature for 24 hours to obtain an n-hexane extract 3. The supernatant was collected by the gradient method, then combined with the n-hexane extracts 1 and 2, and the mixture was filtered with suction using the qualitative filter paper (No. 2 manufactured by ADVANTEC Corporation) to obtain a total n-hexane extract. An acetone extract 1 was obtained by adding 100 mL of acetone to the extraction residue and allowing it to stand at room temperature for 24 hours. The supernatant was collected by the gradient method, 100 mL of acetone was then added to the extraction residue, and the mixture was allowed to stand at room temperature for 24 hours to obtain an acetone extract 2. The supernatant was collected by the gradient method, 100 mL of acetone was then added again, and the mixture was allowed to stand at room temperature for 24 hours to obtain an acetone extract 3. The supernatant was collected by the gradient method and then combined with the acetone extracts 1 and 2, and the mixture was filtered with suction using the qualitative filter paper (No. 2 manufactured by ADVANTEC Corporation) to obtain a total acetone extract.

A chloroform/methanol extract 1 was obtained by adding 50 mL of chloroform and 50 mL of methanol to the extraction residue and allowing the mixture to stand at room temperature for 24 hours. The supernatant was collected by the gradient method, 50 mL of chloroform and 50 mL of methanol were then added to the extraction residue, and the mixture was allowed to stand at room temperature for 24 hours to obtain a chloroform/methanol extract 2. The supernatant was collected by the gradient method, 50 mL of chloroform and 50 mL of methanol were then added again, and the mixture was allowed to stand at room temperature for 24 hours to obtain a chloroform/methanol extract 3. The supernatant was collected by the gradient method, then combined with the chloroform/methanol extracts 1 and 2, and the mixture was filtered with suction using the qualitative filter paper (No. 2 manufactured by ADVANTEC Corporation) to obtain a total chloroform/methanol extract. A hot water extract 1 was obtained by adding 100 mL of a purified water to the extraction residue to obtain a mixture and heating the mixture at 100°C for 24 hours. The supernatant was collected by the gradient method, 100 mL of a purified water was then added to the extraction residue, and the mixture was heated at 100°C for 24 hours to obtain a hot water extract 2. The supernatant was collected by the gradient method, 100 mL of a purified water was then added again, and heated at 100°C for 24 hours to obtain a hot water extract 3. The supernatant was collected by the gradient method, then combined with the hot water extracts 1 and 2, and the mixture was filtered with suction using the qualitative filter paper (No. 2 manufactured by ADVANTEC Corporation) to obtain a total hot water extract. The total n-hexane extract, the total acetone extract, the total chloroform/methanol extract, and the total hot water extract were concentrated with the solvent concentrator (EYELA DPE-1150, manufactured by TOKYO RIKAKIKAI CO., LTD.) and the rotary evaporator (EYELA N-1210BVF-W, manufactured by TOKYO RIKAKIKAI CO., LTD.), respectively. The concentrated extract was freeze-dried in the freeze-dryer (EYELA FDU-1200, TOKYO RIKAKIKAI) to obtain powders of an n-hexane extract, an acetone extract, a chloroform/methanol extract, and a hot water extract, respectively.

The resulting powder of the n-hexane extract had a dry weight of 85.0 mg and a yield of 1.8% with respect to 4.8 g of the dried fruiting body. The resulting powder of the acetone extract had a dry weight of 63.6 mg and a yield of 1.3% with respect to 4.8 g of the dried fruiting body. The resulting powder of the chloroform/methanol extract had a dry weight of 1.8 g and a yield of 38% with respect to 4.8 g of the dried fruiting body. The resulting powder of the hot water extract had a dry weight of 1.1 g and a yield of 23% with respect to 4.8 g of the dried fruiting body.

### (Test Example 1-1. Examination for effects of Cordyceps militaris derived from Samia cynthia ricini in male menopausal model rat)

As the human menopause model, rats were castrated (orchiectomy). An eight-week-old male rat was subjected to orchiectomy under isoflurane anesthesia, and the castrated rat was orally administered the hot water extract of Cordyceps militaris derived from Samia cynthia ricini (RK: 20 mg/0.2 mL distilled water/rat: average body weight at the start of administration: 317 g) together with testosterone propionate (TP: 1 mg/kg, every other day administration) continuously for 12 days. Animals were sacrificed the day after the final treatment, and then the serum, testes, prostate, and seminal vesicle glands were isolated. Some animals to which 0.2 mL distilled water was administered was used as control group.

After the blood collection, the concentrations of testosterone and dihydrotestosterone in the serum were evaluated by the following measurement kit.

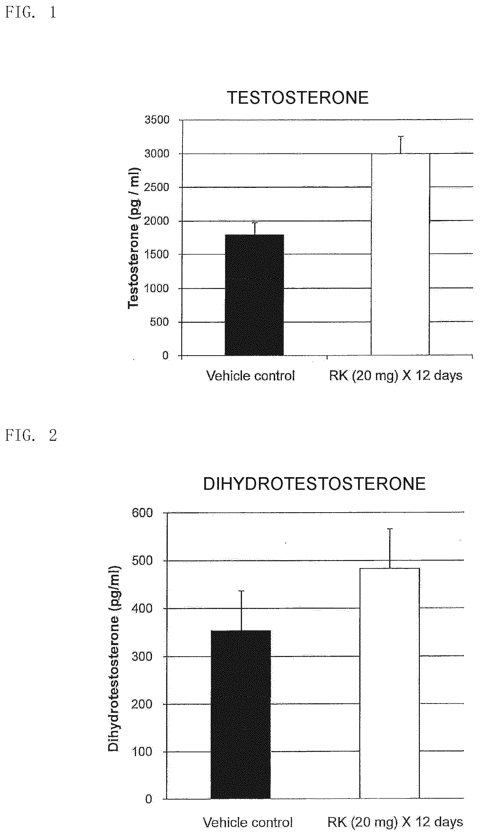

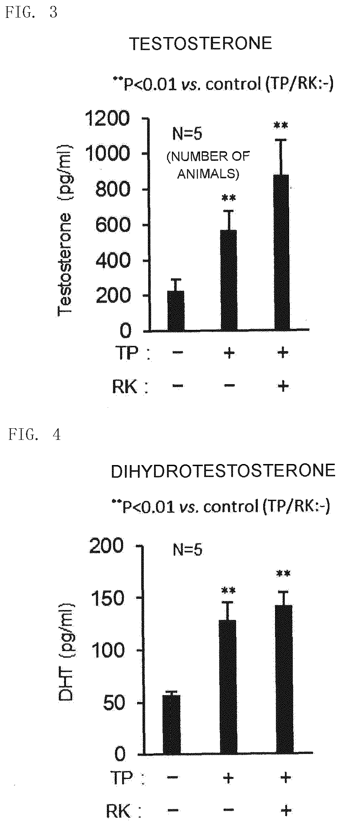

The results of the blood testosterone values measured by an EIA kit (manufactured by Cayman) and the results of blood dihydrotestosterone values measured by an ELISA kit (manufactured by Abnova) are shown in Figs. 1 and 2, respectively. In order to confirm the reproducibility, the results of the retest performed with the same test content are shown in Figs. 3 and 4.

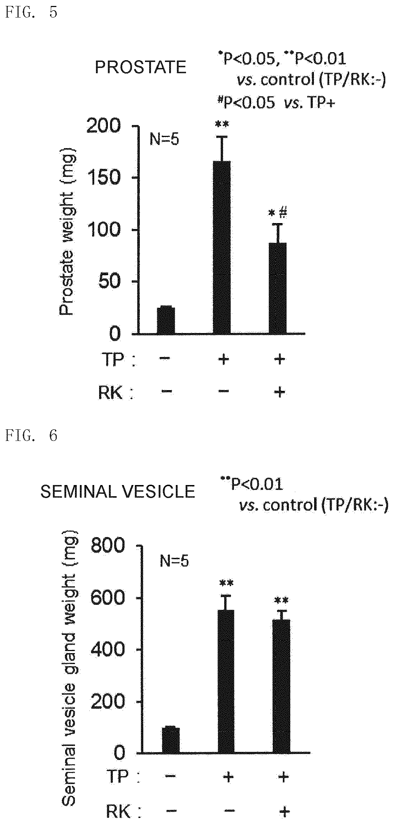

After the autopsy, changes in the weight of the prostate and the seminal vesicle were evaluated. The change in the weight of the prostate is shown in Fig. 5, and change in the weight of the seminal vesicle is shown in Fig. 6.

As shown in the results of Figs. 1 to 4, the serum levels of testosterone and the active metabolite dihydrotestosterone tended to increase by the administration of the hot water extract of Cordyceps militaris derived from Samia cynthia ricini. In this evaluation system, since the testis is excised, endogenous testosterone cannot be newly generated in the body of the rat. It was presumed that by administering the hot water extract of Cordyceps militaris derived from Samia cynthia ricini, testosterone administered as testosterone propionate and dihydrotestosterone, which is an active substance changed in the body, are maintained in the body without being decreased by catabolism (decomposition).

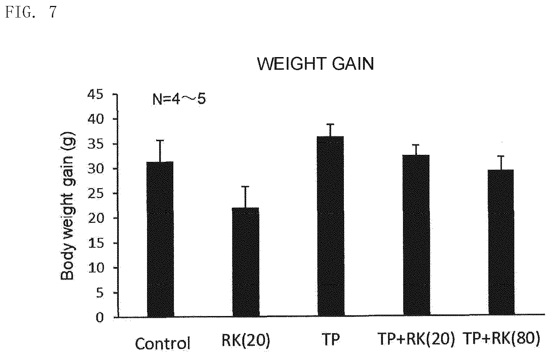

In addition, as shown in the results of Figs. 5 to 6, the weight of the prostate and the seminal vesicle that had atrophied by castration were increased by the administration of testosterone propionate (TP group). Administration of the hot water extract of Cordyceps militaris derived from Samia cynthia ricini did not affect the seminal vesicle weight and size, but significantly reduced the weight of the prostate (TP + RK group). The weight gain after the treatment is shown in Fig. 7. As shown in Fig. 7, it was found that the body weight tended to increase after administration of testosterone propionate to the castrated animals, and in the group treated with Cordyceps militaris derived from Samia cynthia ricini (RK : 20 and 80 mg), the amount of weight gain was lower than that of the TP group, meaning that the rats administered RK exhibits may be less likely to increase their body weight. In addition, the single administration group of Cordyceps militaris derived from Samia cynthia ricini (RK: 20 mg) also showed the tendency to decrease, as compared with control group (Control). There was no difference in body weight between both groups before the start of administration.

### (Test Example 1-2. Examination for effects of Cordyceps militaris derived from Samia cynthia ricini in prostatic hypertrophy model rat)

In a rat model of menopause Test Example 1-1, since the increase in the prostatic weight by testosterone propionate (TP) was inhibited by the hot water extract of Cordyceps militaris derived from Samia cynthia ricini (RK), it was assumed that RK has inhibitory effects on prostatic hypertrophy. The effect of long-term administration of RK was, therefore, examined using a rat model of prostatic hypertrophy in which an excessive amount of TP is administered to mature rats for a long period of time.

An eight-week-old male Wistar rat was orally administered RK (10 mg/0.2 mL distilled water/rat or 20 mg/0.2 mL distilled water/rat: average body weight at the start of administration: 268 g) together with 3 mg/kg of TP continuously for 1 month. Animals were sacrificed the day after the final treatment, and then the serum, testes, prostate, and seminal vesicle glands were isolated. Some animals to which 0.2 mL distilled water was administered was used as control group.

After the blood collection, the serum levels of testosterone and dihydrotestosterone value were evaluated by the following measurement kit.

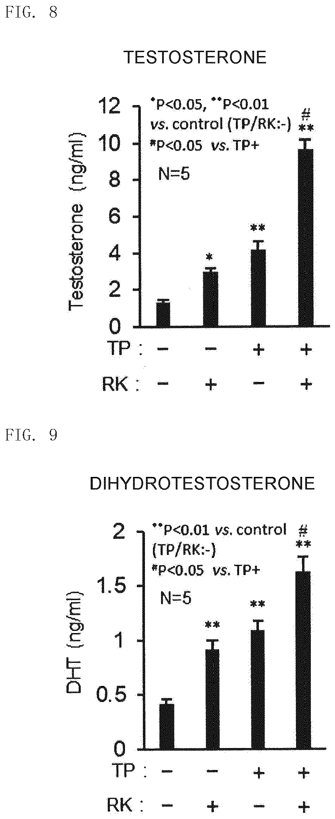

The results of the blood testosterone values measured by EIA kit (manufactured by Cayman) and the results of dihydrotestosterone values measured by Elisa kit (manufactured by Abnova) are shown in Figs. 8 and 9, respectively.

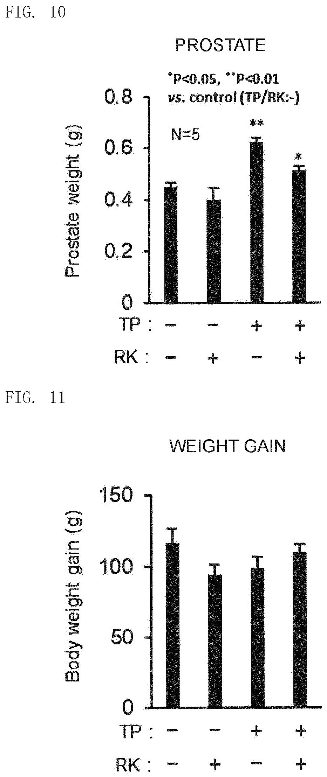

After the necropsy, changes in prostate weight and body weight were evaluated. The change in prostate weight is shown in Fig. 10 and the amount of weight gain is shown in Fig. 11.

As shown in the results of Figs. 8 and 9, relative to the increase in the serum levels of testosterone and dihydrotestosterone by TP treatment, RK administration further significantly enhanced their levels (Tukey-Kramer multiple comparison test). As shown in Fig. 10, the prostatic weight was increased by TP and tended to decrease by RK. As shown in Fig. 11, it was confirmed that the amount of weight gain tended to decrease by long-term administration of RK alone. In the long-term administration examination of Test Example 1-2, it was confirmed that the increase in serum testosterone and dihydrotestosterone concentrations, the decrease in the weight of the prostate, and less gain of the body weight induced by RK was be the same as in Test Example 1-1.

An acute toxicity test (RK: single administration of 1000 mg/mL) was also performed on male Wistar rats, and there was no abnormality in appearance or behavior, no case of death, and no change in male hormone level was observed during 2 weeks of follow-up.

### (Test Example 2. Influence of active ingredient on endocrinology and function of cultured cells from reproductive organs)

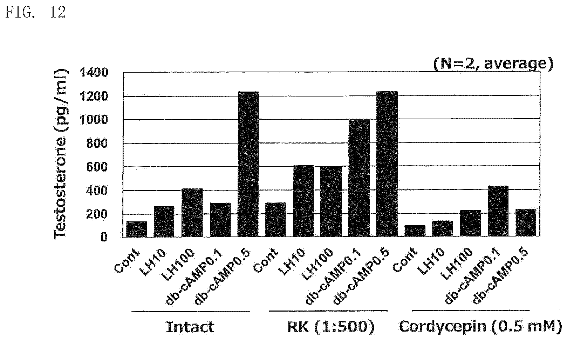

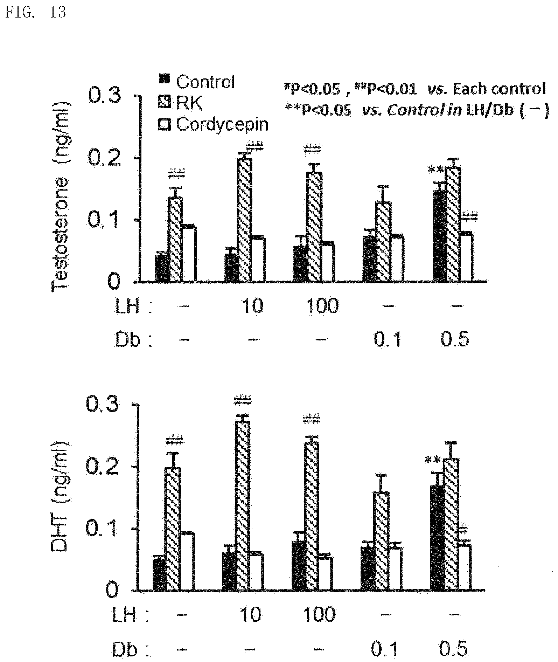

Dispersed testicular cells were prepared from the testis in mature rats. The testicular cells isolated were cultured for 18 hours in DMEM/Ham's F-12 medium (manufactured by FUJIFILM Wako Pure Chemical Corporation) for culture of testicular stromal cells, Leydig cell. Cells were pretreated with the hot water extract of Cordyceps militaris derived from Samia cynthia ricini (diluted to 1:500), or cordycepin known as one of the physiological activity substances of a vegetative wasp (0.5 mM) for 1 hour and then stimulated with a luteinizing hormone (LH: 10 and 100 ng/mL) or dibutyryl cAMP (0.1 and 0.5 mM). Ninety minutes after the stimulation, the media was collected and the content of testosterone was quantified. The results are shown in Fig. 12. In order to confirm the reproducibility, the results of the reexamination performed with the same test content are shown in Fig. 13. In the reexamnination, three independent experiments were performed, and in addition to testosterone, dihydrotestosterone was also quantified.

As shown in the results of Fig. 12 and Fig. 13, when the cells were treated with the hot water extract of Cordyceps militaris derived from Samia cynthia ricini (RK in the drawing), the levels of testosterone and dihydrotestosterone were higher in all groups except for the 0.5 mM dibutyryl cAMP group (discribed as db-cAMP in Fig. 12 and Db in Fig. 13). No stimulatory action such as RK was observed with treatment with Cordycepin at the dose of 0.5 mM. Thus, it was indicated that the hot water extract of Cordyceps militaris derived from Samia cynthia ricini also has a direct stimulatory action on the production of male hormones from the testicular cells.

### (Test Example 3. Effect on proliferation of prostate cancer)

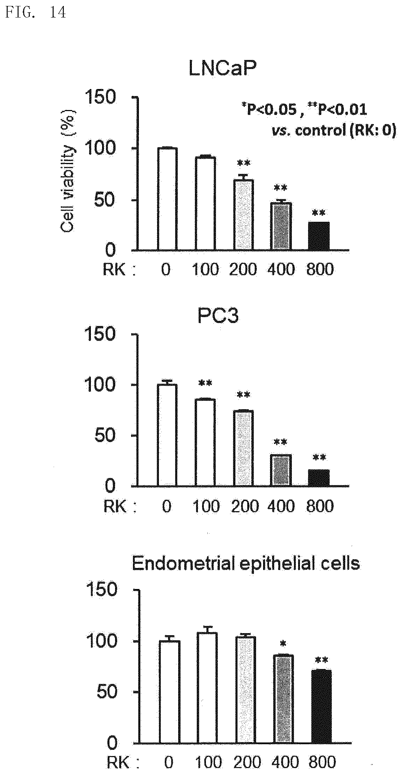

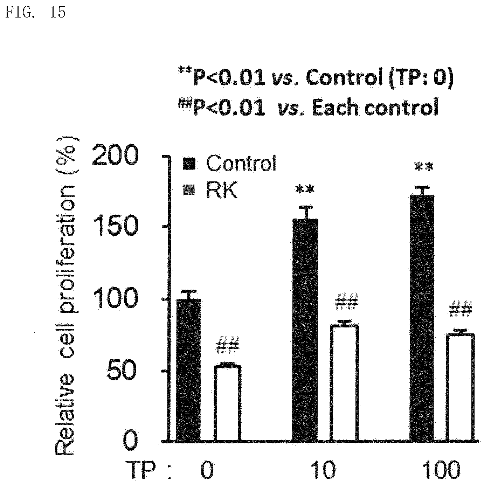

Using the culture system of human prostate cancer cell lines (PC3, LNCaP), the hot water extract of Cordyceps militaris derived from Samia cynthia ricini was applied and cultured for 48 hours, and cell proliferation (cell viability) was evaluated by WST-8 assay (manufactured by DOJINDO LABORATORIES). In addition, human endometrial glandular epithelial cell line was used as a comparative normal control cell, comparing with cancer cells. The results of the effect of the hot water extract of Cordyceps militaris derived from Samia cynthia ricini on cell proliferation are shown in Figs. 14 and 15.

As shown in the results of Fig. 14, the hot water extract of Cordyceps militaris derived from Samia cynthia ricini inhibited proliferation of testosterone insensitive PC3 and testosterone sensitive LNCaP cells in a dose-dependent manner. In PC3 cells, the hot water extract of Cordyceps militaris derived from Samia cynthia ricini (800 µg/mL) showed the cytostatic activity with 80% or more inhibitory effects, compared to control levels, although the extract did not significantly affect the proliferation of the normal epithelial cell. Fig. 15 shows that the cell proliferation in the presence of testosterone (10 and 100 nM) is also inhibited by the hot water extract of Cordyceps militaris (200 µg/mL). Based upon these results, it was inferred that the fruiting body (Cordyceps militaris) derived from Samia cynthia ricini component contains the compound(s) that inhibits the proliferation of human prostatic cells and prostate cancers.

## Claims

1. A composition for inhibiting a decrease in testosterone and/or dihydrotestosterone, the composition comprising Cordyceps militaris derived from Samia cynthia ricini and/or its extract.

2. A composition for preventing and/or treating menopausal disorders, prostatic hypertrophy, a prostate cancer, or infertility, the composition comprising Cordyceps militaris derived from Samia cynthia ricini and/or its extract.

3. A food composition for improving a menopausal condition, a prostatic hypertrophy condition, or an infertility condition, the composition comprising Cordyceps militaris derived from Samia cynthia ricini and/or its extract.

4. The composition of claim 2 or 3, wherein the menopausal disorder or condition is accompanied by at least one condition selected from the group consisting of sexual ability decrease in muscle strength, fatigue, insomnia, joint pain, muscle pain, depression, menstrual abnormality, a decrease in libido, and a decrease in sexual ability.

5. The composition according to any one of claims 1 to 4, wherein the Cordyceps militaris derived from Samia cynthia ricini is obtained within 90 days from hyphal generation.

6. The composition according to any one of claims 1 to 5, wherein the composition is for oral use.

7. The composition according to any one of claims 1 to 6, wherein the composition is a food and drink, a quasi-pharmaceutical product, or a pharmaceutical product.
